# EUROPEAN PATENT APPLICATION

(11) **EP 3 223 252 A1**
(43) Date of publication of application: **27.09.2017**
(21) Application number: 17161373.0
(22) Date of filing: 16.03.2017
(51) Int. Cl.: G08B 13/19, G08B 21/04

(54) **SYSTEM AND METHOD FOR MONITORING ABNORMAL BEHAVIOR**

(30) Priority: 25.03.2016 CN 201610174919
(71) Applicant: Chiun Mai Communication Systems, Inc., New Taipei City 236 (TW)
(72) Inventor: WU, KUANG-HUI, New Taipei, Taiwan (CN); CHEN, HUANG-MU, New Taipei, Taiwan (CN)
(74) Representative: Cordina, Kevin John

(57) **Abstract**

A system for monitoring abnormal behavior includes a distribution sensing unit and a controlling unit. The controlling unit is electrically connected to the distribution sensing unit. The distribution sensing unit is configured to sense a change of a temperature of a target object in the monitoring area according to a background temperature value and output distribution data of the temperature change. The controlling unit is configured to receive and analyze the distribution data of the temperature change to identify a behavior of the target object.

## Description

### CROSS-REFERENCE TO RELATED APPLICATIONS

This application claims priority to Chinese Patent Application No. 201610174919.1 filed on March 25, 2016, the contents of which are incorporated by reference herein.

### FIELD

The subject matter herein generally relates to a system and method for monitoring abnormal behavior.

### BACKGROUND

In general, there are two ways for monitoring people's behavior. A first way is identifying people's behavior through a camera combined with back-end image analysis. A second way is monitoring people's behavior through a wearable device worn on a user. However, the first way is likely to involve the infringement of personal privacy. In the second way, once the user don't want to wear the wearable device, it may be unable and inconvenient to monitor people's behavior.

### SUMMARY

A system for monitoring abnormal behavior includes a distribution sensing unit and a controlling unit. The controlling unit is electrically connected to the distribution sensing unit. The distribution sensing unit is configured to sense a change of a temperature of a target object in the monitoring area according to a background temperature value and output distribution data of the temperature change. The controlling unit is configured to receive and analyze the distribution data of the temperature change to identify a behavior of the target object. The system for monitoring abnormal behavior can be conveniently used whenever and wherever possible.

### BRIEF DESCRIPTION OF THE DRAWINGS

Implementations of the present technology will now be described, by way of example only, with reference to the attached figures.
FIG. 1 is a block diagram of an exemplary embodiment of a system for monitoring abnormal behavior.
FIG. 2 is a flowchart of a method for monitoring abnormal behavior.

### DETAILED DESCRIPTION

It will be appreciated that for simplicity and clarity of illustration, where appropriate, reference numerals have been repeated among the different figures to indicate corresponding or analogous elements. In addition, numerous specific details are set forth in order to provide a thorough understanding of the embodiments described herein. However, it will be understood by those of ordinary skill in the art that the embodiments described herein can be practiced without these specific details. In other instances, methods, procedures, and components have not been described in detail so as not to obscure the related relevant feature being described. Also, the description is not to be considered as limiting the scope of the embodiments described herein. The drawings are not necessarily to scale and the proportions of certain parts have been exaggerated to better illustrate details and features of the present disclosure.

Several definitions that apply throughout this disclosure will now be presented.

The term "substantially" is defined to be essentially conforming to the particular dimension, shape, or other feature that the term modifies, such that the component need not be exact. For example, substantially cylindrical means that the object resembles a cylinder, but can have one or more deviations from a true cylinder. The term "comprising," when utilized, means "including, but not necessarily limited to"; it specifically indicates open-ended inclusion or membership in the so-described combination, group, series and the like.

The present disclosure is described in relation to a system and a method for monitoring abnormal behavior.

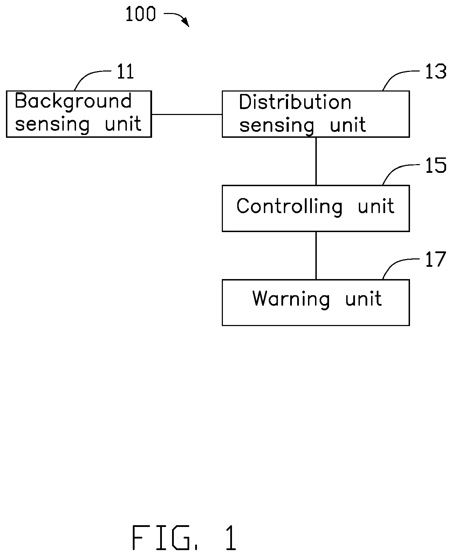

FIG. 1 illustrates a system 100 for monitoring abnormal behavior, which can be implemented inside a building, such as a shopping mall, a factory, a hospital, a hotel, a restaurant, an airport, and so on. The system 100 is used to monitor whether a target object, such as, a staff or an old man has an abnormal behavior. Once the behavior of the target object is determined to be abnormal, the system 100 is further used to generate a warning.

The system 100 includes a background sensing unit 11, a distribution sensing unit 13, a controlling unit 15, and a warning unit 17. The background sensing unit 11 and the distribution sensing unit 13 can be placed on a wall or a ceiling of the building.

In this exemplary embodiment, the background sensing unit 11 includes a temperature sensor. The background sensing unit 11 is configured to sense a temperature of a monitoring area and generate a corresponding sensing signal.

In this exemplary embodiment, the distribution sensing unit 13 includes integrated infrared sensors arranged in an N*N matrix array. N is a predefined positive integer, for example, 8, 10 or 12. The distribution sensing unit 13 is electrically or wirelessly connected to the background sensing unit 11. The distribution sensing unit 13 is configured to receive the sensing signal from the background sensing unit 11. The sensing signal is the temperature data of the monitoring area sensed by the background sensing unit 11. The distribution sensing unit 13 is further configured to adjust a background temperature value according to the received temperature data. When a target object having a temperature different from the background temperature value, for example, a staff enters the monitoring area, the distribution sensing unit 13 senses a change of the temperature with different degrees and outputs distribution data of the temperature change to the controlling unit 15.

In this exemplary embodiment, when the distribution sensing unit 13 senses a temperature of the target object, the background temperature value sensed by the background sensing unit 11 will affect the sensing of the distribution sensing unit 13. Thus, when the distribution sensing unit 13 receives the temperature data from the background sensing unit 11, the distribution sensing unit 13 will adjust the background temperature value according to the temperature data from the background sensing unit 11 to improve an accuracy of the sensed results. In other words, the background sensing unit 11 is mainly configured to provide the background temperature value of the monitoring area and the background temperature value is served as an input value of the distribution sensing unit 13. The distribution sensing unit 13 can automatically makes a response to a change of the environment or the monitoring area, then obtain a correct temperature distribution diagram.

In this exemplary embodiment, due to the distribution sensing unit 13 is an N*N matrix array of integrated infrared sensors, the temperature distribution diagram may include N*N pixels. Then, the distribution sensing unit 13 has a low resolution and may not to involve the infringement of personal privacy.

In other exemplary embodiments, the background sensing unit 11 includes a humidity sensor. The background sensing unit 11 is configured to sense a humidity of the monitoring area and generate a humidity signal. In other exemplary embodiments, the background sensing unit 11 includes a temperature sensor and a humidity sensor. The background sensing unit 11 is configured to sense a temperature and a humidity of the monitoring area and generate a temperature signal and a humidity signal. In other words, the background sensing unit 11 can include a temperature sensor and/or a humidity sensor. The background sensing unit 11 is configured to sense a temperature and/or a humidity of the monitoring area and generate a temperature signal and/or a humidity signal. The distribution sensing unit 13 is configured to receive the sensing signal from the background sensing unit 11, that is, the temperature data and/or humidity data sensed by the background sensing unit 11. The distribution sensing unit 13 is further configured to adjust the background temperature value according to the temperature data and/or humidity data.

In another exemplary embodiment, the background sensing unit 11 may be omitted. The background temperature value is fixed. The distribution sensing unit 13 does not adjust the background temperature value.

The controlling unit 15 can be a personal computer or a microprocessor. The controlling unit 15 is configured to receive the sensing data from the distribution sensing unit 13. For example, the sensing data may be the temperature distribution diagram. The controlling unit 15 is further configured to analysis the sensing data from the distribution sensing unit 13 through algorithm or other method to identify the behavior of the target object. For example, according to the sensing data of the distribution sensing unit 13, that is, the temperature distribution diagram, the controlling unit 15 can determine that the target object is in a walking mode, a lying mode, or a stand-up mode. When the target object enters the monitoring area, the controlling unit 15 can further determine a walking direction of the target object according to the temperature distribution diagram obtained by the distribution sensing unit 13.

The controlling unit 15 is further configured to determine whether the behavior of the target object is abnormal. For example, when the behavior of an old man is from the stand-up mode to the lying mode, the controlling unit 15 can determine that the target object may be fell down.

When the target object enters the monitoring area, the controlling unit 15 is further configured to determine whether the behavior of the target object is a prohibited behavior according to the walking direction of the target object. For example, when the target object walks towards a prohibited area, the controlling unit 15 can determine that the behavior of the target object is a prohibited behavior. In other exemplary embodiments, when a time of the target object staying in one area is greater than a predetermined time, the controlling unit 15 can also determine that the behavior of the target object is an abnormal behavior.

The controlling unit 15 is electrically connected to the warning unit 17. When the controlling unit 15 determines that the target object has an abnormal behavior, the controlling unit 15 is configured to output a warning signal to the warning unit 17. In this exemplary embodiment, when the warning unit 17 receives the warning signal, the warning unit 17 determines related people who can receive the warning signal according to a type of the abnormal behavior. The warning unit 17 further sends the warning signal to the related people through wireless or wired network. For example, when the controlling unit 15 determines that an old man falls down, the warning unit 17 determines that a family member of the old man can receive the warning signal and sends the warning signal to the family member of the old man. When the controlling unit 15 determines that a target object, for example, a staff, walks towards a prohibited area, the warning unit 17 determines that a person in charge or other people can receive the warning signal and sends the warning signal to the person in charge and the target object.

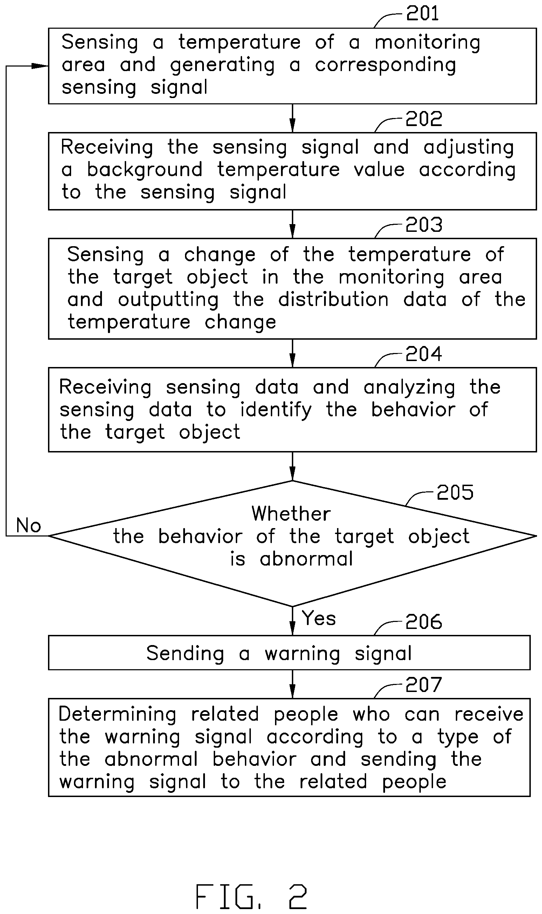

FIG. 2 illustrates a flowchart of a method for monitoring abnormal behavior. The method is provided by way of example, as there are a variety of ways to carry out the method. Each block shown in FIG. 2 represents one or more processes, methods, or subroutines which are carried out in the example method. Furthermore, the order of blocks is illustrative only and additional blocks can be added or fewer blocks may be utilized without departing from the scope of this disclosure.

At block 201, the background sensing unit 11 senses a temperature of a monitoring area and generates a corresponding sensing signal.

At block 202, the distribution sensing unit 13 receives the sensing signal from the background sensing unit 11 and adjusts a background temperature value according to the sensing signal. The sensing signal is the temperature data of the monitoring area sensed by the background sensing unit 11.

In this exemplary embodiment, the background sensing unit 11 includes a temperature sensor and/or a humidity sensor. The background sensing unit 11 is configured to sense a temperature and/or a humidity of the monitoring area and generate a temperature signal and/or a humidity signal. The distribution sensing unit 13 is configured to receive the sensing signal from the background sensing unit 11, that is, the temperature data and/or humidity data sensed by the background sensing unit 11. The distribution sensing unit 13 is further configured to adjust the background temperature value according to the temperature data and/or humidity data.

At block 203, the distribution sensing unit 13 senses a change of the temperature of the target object in the monitoring area and outputs the distribution data of the temperature change to the controlling unit 15.

In this exemplary embodiment, the distribution sensing unit 13 includes integrated infrared sensors arranged in an N*N matrix array. N is a predefined positive integer, for example, 8, 10 or 12. The background sensing unit 11 is mainly configured to provide the background temperature value of the monitoring area and the background temperature value is served as an input value of the distribution sensing unit 13. The distribution sensing unit 13 can automatically makes a response to a change of the environment or the monitoring area, then obtain a correct temperature distribution diagram.

In this exemplary embodiment, due to the distribution sensing unit 13 is an N*N matrix array of integrated infrared sensors, the temperature distribution diagram may include N*N pixels. Then, the distribution sensing unit 13 has a low resolution and may not to involve the infringement of personal privacy.

At block 204, the controlling unit 15 receives the sensing data from the distribution sensing unit 13. For example, the sensing data may be the temperature distribution diagram. The controlling unit 15 further analyses the sensing data through algorithm or other method to identify the behavior of the target object.

The controlling unit 15 can be a personal computer or a microprocessor.

At block 205, the controlling unit 15 determines whether the behavior of the target object is abnormal. When the controlling unit 15 determines that the behavior of the target object is normal, block 201 is implemented. That is, the background sensing unit 12 continues to sense a temperature of the monitoring are. When the controlling unit 15 determines that the behavior of the target object is abnormal, block 206 is implemented.

At block 206, the controlling unit 15 outputs a warning signal to the warning unit 17.

At block 207, the warning unit 17 determines related people who can receive the warning signal according to a type of the abnormal behavior and sends the warning signal to the related people through wireless or wired network.

For example, when the controlling unit 15 determines that an old man fell down, the warning unit 17 determines that family members of the old man can receive the warning signal and sends the warning signal to the family members of the old man. When the controlling unit 15 determines that the target object, for example, a staff, walks towards the prohibited area, the warning unit 17 determines that a person in charge or other people can receive the warning signal and sends the warning signal to the person in charge and the target object.

In another exemplary embodiment, the background sensing unit 11, block 201 and block 202 may be omitted. The background temperature value is fixed. The distribution sensing unit 13 does not adjust the background temperature value.

The system for monitoring abnormal behavior can be conveniently used whenever and wherever possible. Furthermore, the system 100 need not to wear a wearable device and also will not involve the infringement of personal privacy. The system 100 can be convenient to monitor people's behavior, and once a behavior of a target object is determined to be abnormal, the system 100 can timely generate a warning to the related people through the warning unit 17.

The exemplary embodiments shown and described above are only examples. Many details are often found in the art such as the other features of the system and method. Therefore, many such details are neither shown nor described. Even though numerous characteristics and advantages of the present technology have been set forth in the foregoing description, together with details of the structure and function of the present disclosure, the disclosure is illustrative only, and changes may be made in the details, especially in matters of shape, size and arrangement of the parts within the principles of the present disclosure up to, and including the full extent established by the broad general meaning of the terms used in the claims. It will therefore be appreciated that the embodiments described above may be modified within the scope of the claims.

## Claims

1. A system for monitoring abnormal behavior, comprising:
a distribution sensing unit configured to sense a change of a temperature of a target object in a monitoring area according to a background temperature value and output distribution data of the temperature change; and
a controlling unit electrically connected to the distribution sensing unit, the controlling unit configured to receive and analyze the distribution data of the temperature change to identify a behavior of the target object.

2. The system of claim 1, further comprising:
a background sensing unit electrically or wirelessly connected to the distribution sensing unit and configured to sense the monitoring area and generate a sensing signal, wherein the distribution sensing unit is further configured to receive the sensing signal and adjust the background temperature value according to the sensing signal.

3. The system of claim 2, wherein the background sensing unit comprises a temperature sensor, the background sensing unit senses a temperature of the monitoring area, the distribution sensing unit receives temperature data in the monitoring area sensed by the background sensing unit and adjusts the background temperature value according to the temperature data.

4. The system of claim 2, wherein the background sensing unit comprises a humidity sensor, the background sensing unit senses a humidity of the monitoring area, the distribution sensing unit receives humidity data in the monitoring area sensed by the background sensing unit and adjusts the background temperature value according to the humidity data.

5. The system of claim 2, wherein the background sensing unit comprises a temperature sensor and a humidity sensor, the background sensing unit senses a temperature and a humidity of the monitoring area, the distribution sensing unit receives temperature data and humidity data in the monitoring area sensed by the background sensing unit and adjusts the background temperature value according to the temperature data and humidity data.

6. The system of claim 1, wherein the distribution sensing unit comprises integrated infrared sensors arranged in an N*N matrix array, N is a predefined positive integer.

7. The system of claim 1, wherein the controlling unit is further configured to determine whether the behavior of the target object is abnormal, when the behavior of the target object is determined to be abnormal, the controlling unit further outputs a warning signal.

8. The system of claim 7, further comprising a warning unit, wherein the warning unit is electrically connected to the controlling unit, the warning unit is configured to determine related people who can receive the warning signal according to a type of the abnormal behavior and sends the warning signal to the related people.

9. A method for monitoring abnormal behavior, the method comprising:
(a) sensing a change of a temperature of a target object in a monitoring area according to a background temperature value and outputting distribution data of the temperature change; and
(b) analyzing the distribution data of the temperature change of the target object in the monitoring area to identify a behavior of the target object.

10. The method of claim 9, further comprising:
(c) sensing the monitoring area and generating a sensing signal; and
(d) adjusting the background temperature value according to the sensing signal.

11. The method of claim 10, wherein step (c) further comprises:
sensing a temperature and/or a humidity of the monitoring area and generating the sensing signal.

12. The method of claim 9, further comprising:
determining whether the behavior of the target object is abnormal; and
outputting a warning signal when the behavior of the target object is determined to be abnormal.

13. The method of claim 12, wherein when the behavior of the target object is determined to be abnormal, the method further comprises:
determining related people who can receive the warning signal according to a type of the abnormal behavior and sending the warning signal to the related people.
